# FASCICULE DE BREVET EUROPEEN

(11) **EP 4 040 195 B1**
(45) Date de publication et mention de la délivrance du brevet: **13.05.2026**
(21) Numéro de dépôt: 22155680.6
(22) Date de dépôt: 08.02.2022
(51) Int. Cl.: G01T 1/20

(54) **CASSETTE RADIOLOGIQUE AVEC ABSORBEURS DE CHOC**
RÖNTGENKASSETTE MIT DÄMPFUNGSMITTELN
X-RAY CASSETTE WITH SHOCK ABSORBERS

(30) Priorité: 08.02.2021 FR 2101172
(43) Date de publication de la demande: 10.08.2022
(73) Titulaire: Trixell, 38430 Moirans (FR)
(72) Inventeur: PONS, Sylvain, 38430 MOIRANS (FR); RIEUVERNET, Pierre, 38430 MOIRANS (FR)
(74) Mandataire: Atout PI Laplace

(56) Documents cités:
- JP-A- 2009 257 914
- JP-A- 2016 033 516
- US-A1- 2020 333 483

## Description

L'invention se situe dans le domaine de l'imagerie. Elle peut être appliquée à tout type d'imageur, notamment les imageurs à rayons X, visibles, infrarouges. L'invention est explicitée ici dans le domaine de l'imagerie médicale par rayons X, ceci à titre d'exemple et sans perte d'applicabilité aux autres domaines d'imagerie. L'invention concerne une cassette radiologique portable avec absorbeurs de choc améliorant la protection de la cassette contre les chutes, impacts d'objets extérieurs, efforts de pression localisés ou répartis, et sollicitations quelconques.

L'invention se réfère à une cassette radiologique portable telle que définie dans la revendication 1.

La cassette comprend un détecteur numérique de rayonnement ionisant permettant de fournir une image fonction du rayonnement reçu. Le système radiologique comprend en outre une source de rayonnement ionisant, comme par exemple un tube à rayons X, permettant de générer un rayonnement X et une station de base comprenant un système de traitement de l'information permettant de synchroniser le tube à rayon X et le détecteur et permettant aussi de réaliser des traitements d'images comme de présenter à l'opérateur l'image corrigée de tous les défauts inhérents au détecteur et améliorée, par exemple par des traitements de rehaussement de contour. Un objet dont on veut obtenir l'image X est placé entre la source et le détecteur. Un tel système peut être utilisé dans de nombreuses applications telles que par exemple la radiologie médicale et le contrôle non destructif. L'invention peut également être mise en œuvre pour d'autres types de rayonnements à détecter notamment des rayonnements gamma.

Par le passé, les systèmes radiologiques comprenant des capteurs digitaux ou des tubes intensificateurs d'image étaient volumineux et peu mobiles. Il était nécessaire de positionner l'objet par rapport au système pour obtenir l'image désirée. Avec l'apparition de nouvelles générations de détecteurs à l'état solide, le détecteur est devenu moins volumineux et il a été possible de déplacer le détecteur par rapport à un objet restant fixe. Pour la radiologie médicale, on a réalisé des détecteurs numériques sous forme de cassettes mobiles qu'il devient possible de placer à proximité immédiate d'un patient dont on veut réaliser une image, lorsque l'état de santé du patient empêche son déplacement vers une salle réservée à la radiologie. Ces détecteurs ont désormais des caractéristiques géométriques similaires aux anciens capteurs analogiques de type cassette ou écran électroluminescent. Ils restent toutefois plus fragiles mécaniquement que ces anciens capteurs analogiques et sont en particulier plus sensibles au risques de casse des éléments internes en cas de chute ou de choc violent.

La cassette mobile comprend essentiellement un détecteur numérique de rayonnement ionisant ayant la forme d'un panneau plat et une carte électronique assurant notamment le pilotage du détecteur numérique. Le détecteur et la carte sont disposés dans un boitier assurant leur protection mécanique.

La cassette utilisée dans un système portable subit beaucoup plus de manipulations que dans un système radiologique fixe et il est nécessaire de renforcer sa protection mécanique, notamment vis-à-vis de chocs que la cassette peut être amenée à subir lors de ses déplacements. Plus précisément, le détecteur numérique est souvent réalisé à partir de composant photosensibles disposés en matrice sur une dalle de verre formant l'élément le plus fragile de la cassette. Outre les chocs qui pourraient l'endommager, cette dalle est également sensible aux déformations notamment en torsion.

Les cassettes portables doivent alors concilier une extrême résistance aux agressions extérieures avec une masse et un format réduits. En effet, ces cassettes portables peuvent être exposées lors de leur manipulation, et tout au long de leur vie, à des chutes, impacts d'objets extérieurs, efforts de pression localisés ou répartis, et des sollicitations de flexion lorsque le poids d'un patient s'exerce sur un détecteur qui n'est pas supporté de façon homogène. Pour cela, la structure mécanique des détecteurs doit assurer une protection maximale des éléments fragiles que sont le détecteur numérique et la carte électronique.

Les chocs, vibrations ou jeux mécaniques internes à la cassette résultent en des dégradations induites par ces contraintes mécaniques. Ces dégradations peuvent mener à la casse de la partie active du détecteur (matrice de photodiode sur un substrat de verre, éventuellement encapsulé par un capot de verre situé au-dessus du scintillateur), à la rupture ou l'endommagement des cartes électroniques ou de composants placés sur ces cartes, au détachement ou rupture des connecteurs ou nappes souples destinés à relier électriquement les différents sous-ensembles, et enfin à des perturbations de l'image radiologique causées par la vibration des connecteurs souples (aussi appelés modules flexibles) qui assurent le contact électrique entre la matrice de photodiodes et les cartes électroniques. Ces dégradations peuvent intervenir immédiatement pendant la sollicitation mécanique et créer une défaillance instantanée. Elles peuvent également provenir de mécanismes d'usure liés aux frottements ou à la répétition de petites perturbations pendant toute la durée de vie du produit.

Pour protéger les parties sensibles d'une cassette, plusieurs solutions sont mises en œuvre actuellement. Une première solution consiste à ajouter des couches intégrées à un film extérieur de protection. Cette solution ne protège pas le panneau des chocs internes dus aux mouvements intérieurs lors de la chute et tend à augmenter l'encombrement du produit qui est contraint par le respect des normes dimensionnelles pour les dispositifs radiologiques.

Une autre solution est d'ajouter des équerres locales sur les bords de l'embase. Cette solution ne protège pas les modules (connecteurs souples) contre les vibrations ou les risques d'arrachement. De plus, la capacité d'absorption est très limitée à cause de la petite taille de ces équerres (l'encombrement étant contraint par la taille normalisée des détecteurs).

Enfin il est également connu de réduire les jeux mécaniques. Mais il n'existe pas, à ce jour, de solution simple pour réduire le jeu mécanique hormis de resserrer les tolérances dimensionnelles de toutes les pièces avec un impact sur le coût voire sur la faisabilité de ces pièces et la difficulté de montage. Une cassette est connue de US 2020/333483 A1. radiologique

Toutes ces options n'offrent qu'une protection partielle contre un groupe restreint de sollicitations mécaniques (chocs, vibrations) et ne sont pas entièrement satisfaisantes.

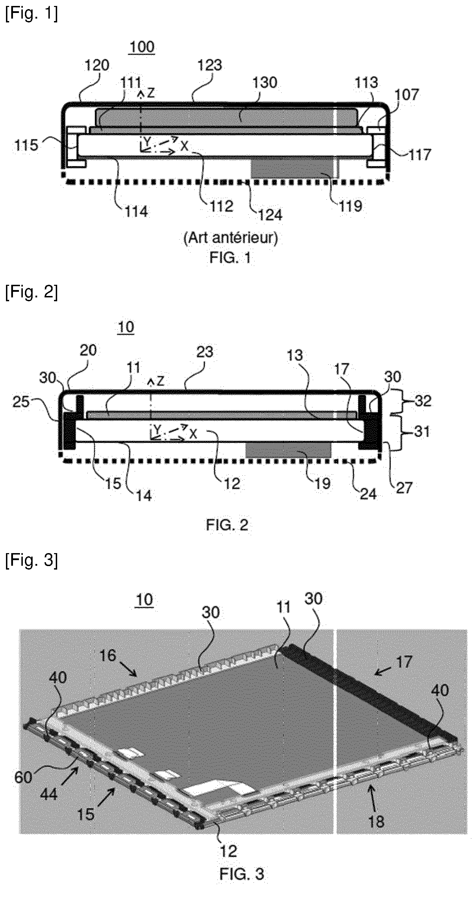

La figure 1 représente une vue en coupe d'une structure de cassette radiologique portable 100 connue de l'art antérieur. Classiquement, une cassette radiologique portable 1 comprend :
- Un détecteur numérique 111 de rayonnement ionisant sous forme d'un panneau plat s'étendant selon un plan (XY);
- Une embase 112 comprenant une première face principale 113, une deuxième face principale 114, opposée à la première face principale 113, l'embase 112 étant délimitée par quatre faces latérales 115, 116 (non représentée car non visible sur la vue en coupe), 117, 118 représentée car non visible sur la vue en coupe), l'embase 112 supportant le détecteur numérique 111 sur la première face principale 113 ;
- une carte électronique 119 assurant la gestion du détecteur numérique 111 ;
- un boîtier 120 de protection mécanique, dans lequel sont disposés l'embase 112, le détecteur numérique 111 et la carte électronique 119, le boîtier 120 comportant quatre faces latérales, une face supérieure 123 et une face inférieure 124.

La cassette radiologique portable 100 de l'art antérieur comprend également deux éléments 107 positionnés à l'intérieur du boîtier 120, chaque élément 107 étant placé contre une face latérale du boîtier et l'embase. Les deux éléments 107 assurent le rôle de tampon contre les chocs dans la direction latérale, c'est-à-dire dans un plan parallèle au plan de l'embase (parallèle au plan (XY). Une couche en mousse 130 est superposée selon l'axe Z perpendiculaire au plan (XY) sur le détecteur numérique, et positionnée entre le détecteur numérique 111 et la face supérieure 123 du boîtier pour isoler le détecteur des chocs selon l'axe Z. Autrement dit, dans une cassette radiologique de l'art antérieur, afin d'isoler le détecteur numérique et autres composants fragiles de la cassette, il est nécessaire d'avoir des éléments d'isolation aux chocs latéraux et une couche de protection additionnelle pour absorber les chocs selon l'axe Z.

Les éléments absorbeurs de chocs 107 visant à absorber les chocs sont montés sur le boîtier et le panneau (ensemble embase et détecteur numérique) est ensuite posé sur le boîtier. Cette disposition ne permet pas de maîtriser les interfaces de transmissions de contraintes entre les éléments absorbeurs de chocs et le panneau. Les éléments absorbeurs de choc ne sont efficaces qu'en cas de sollicitation sur les bords du détecteur et peuvent donc être considérés comme une protection bidimensionnelle. Les chocs en face avant sont uniquement absorbés par la couche de mousse qui filtre très peu les chocs et vibrations, et qui les transmet directement à la dalle sans passer par les éléments absorbeurs de chocs. Une telle cassette radiologique 100 de l'art antérieur est notamment décrite dans le document US 7989773B2.

L'invention vise à pallier tout ou partie des problèmes cités plus haut en proposant une cassette radiologique portable avec absorbeurs de choc permettant sa protection contre les sollicitations mécaniques occasionnelles ou répétées qui risquent de dégrader irréversiblement le produit ou d'altérer ses performances lors d'une utilisation en environnement perturbé. L'invention permet d'améliorer la robustesse mécanique intrinsèque de la cassette en assurant à la fois la protection de la partie active interne du détecteur intégrée dans le boîtier externe lors de chutes du détecteur ou de chocs violents, la protection des composants sensibles lors de l'acquisition d'images générant des défauts images au regard des vibrations, la réduction des jeux mécaniques internes entre le boîtier et l'ensemble formé par l'embase et le détecteur numérique.

A cet effet, l'invention a pour objet une cassette radiologique portable comprenant :
- Un détecteur numérique de rayonnement ionisant sous forme d'un panneau plat ;
- Une embase monobloc comprenant une première face principale, une deuxième face principale, opposée à la première face principale, l'embase étant délimitée par quatre faces latérales, l'embase supportant le détecteur numérique sur la première face principale ;
- une carte électronique assurant la gestion du détecteur numérique ;
- un boîtier de protection mécanique, dans lequel sont disposés l'embase, le détecteur numérique et la carte électronique, le boîtier comportant quatre faces latérales, une face supérieure et une face inférieure;

la cassette radiologique portable comprenant au moins une pièce tridimensionnelle, chacune des au moins une pièce tridimensionnelle étant associée à au moins une des quatre faces latérales de l'embase, chaque pièce tridimensionnelle comprenant :
   - une partie inférieure reliée à l'embase et enveloppant au moins partiellement la au moins une face latérale de l'embase à laquelle la pièce tridimensionnelle est associée;
   - une partie supérieure s'étendant depuis la première face principale de l'embase jusqu'à la face supérieure du boîtier ;
la cassette radiologique portable comprenant un circuit souple, et la partie inférieure d'au moins une des pièces tridimensionnelles comprend au moins un évidement destiné à loger le circuit souple ;la partie supérieure d'au moins une des pièces tridimensionnelles comprenant au moins une ouverture selon un axe sensiblement perpendiculaire à la face latérale de l'embase à laquelle la pièce tridimensionnelle est associée ;
la cassette radiologique portable comprenant un peigne comprenant une branche s'étendant sensiblement parallèlement à la première face principale de l'embase et en contact sur la partie inférieure de la pièce tridimensionnelle, la branche étant pourvue d'au moins une dent s'étendant sensiblement perpendiculairement à la branche , la au moins une dent étant configurée pour coopérer avec la pièce tridimensionnelle de sorte à immobiliser la branche contre le circuit souple.

Avantageusement, la partie inférieure d'au moins une des pièces tridimensionnelles enveloppe partiellement une face latérale adjacente à la face latérale de l'embase à laquelle la pièce tridimensionnelle est associée.

Avantageusement, la au moins une dent est insérée dans l'ouverture de la partie supérieure de la pièce tridimensionnelle et configurée pour bloquer le peigne en translation dans un plan sensiblement parallèle à l'embase.

Avantageusement, la partie supérieure d'au moins une des pièces tridimensionnelles se termine par une forme complémentaire à la face supérieure du boîtier.

Avantageusement, la au moins une pièce tridimensionnelle est en élastomère, en polyuréthane, en élastomère thermoplastique, en polyamide et/ou en polyester.

Avantageusement, la partie inférieure de la au moins une pièce tridimensionnelle est collée à l'embase.

L'invention concerne aussi une pièce tridimensionnelle destinée à coopérer avec :
- une embase monobloc comprenant une première face principale, une deuxième face principale, opposée à la première face principale, l'embase étant délimitée par quatre faces latérales, l'embase étant apte à supporter un détecteur numérique sur la première face principale et une carte électronique,
- un boîtier de protection mécanique, l'embase, le détecteur numérique et la carte électronique étant destinés à être disposés dans le boîtier de protection mécanique, le boîtier comportant quatre faces latérales, une face supérieure et une face inférieure;
   la pièce tridimensionnelle comprenant :
   - une partie inférieure reliée à l'embase et enveloppant au moins partiellement une face latérale de l'embase ;
   - une partie supérieure s'étendant depuis la première face principale de l'embase jusqu'à la face supérieure du boîtier.

L'invention concerne aussi un produit programme d'ordinateur, ledit programme d'ordinateur comprenant des instructions exécutables par ordinateur qui, lorsqu'elles sont exécutées par un processeur, amènent le processeur à commander un appareil de fabrication additive pour fabriquer la pièce tridimensionnelle selon l'invention

L'invention concerne aussi un procédé de fabrication de la pièce tridimensionnelle selon l'invention par fabrication additive, le procédé comprenant: obtenir un fichier électronique représentant une géométrie d'un produit dans lequel le produit est la pièce tridimensionnelle; et commander un appareil de fabrication additive pour fabriquer, sur une ou plusieurs étapes de fabrication additive, le produit selon la géométrie spécifiée dans le fichier électronique.

L'invention sera mieux comprise et d'autres avantages apparaîtront à la lecture de la description détaillée d'un mode de réalisation donné à titre d'exemple, description illustrée par le dessin joint dans lequel :
[Fig.1] la figure 1 représente schématiquement une vue en coupe d'une cassette radiologique de l'art antérieur ;
[Fig.2] la figure 2 représente schématiquement une vue en coupe d'une partie d'une cassette radiologique selon l'invention ;
[Fig.3] la figure 3 représente schématiquement une vue d'une partie de la cassette numérique portable selon l'invention sans boîtier;
[Fig.4] la figure 4 représente schématiquement un agrandissement de la vue de la cassette numérique portable selon l'invention sans boîtier présentée à la figure 3 ;
[Fig.5] la figure 5 représente une pièce tridimensionnelle pour une cassette numérique portable selon l'invention ;
[Fig.6] la figure 6 représente schématiquement une vue d'un autre mode de réalisation de la cassette numérique portable selon l'invention sans boîtier;
[Fig.7] la figure 7 représente schématiquement un agrandissement de la vue de la cassette numérique portable selon l'invention sans boîtier présentée à la figure 6 ;
[Fig.8] la figure 8 représente schématiquement les étapes d'un procédé de fabrication de la pièce tridimensionnelle selon l'invention par fabrication additive.

Sur ces figures, dans un souci de clarté, les échelles ne sont pas respectées. Par ailleurs, les mêmes éléments porteront les mêmes repères dans les différentes figures.

La figure 1 représente schématiquement une vue en coupe d'une cassette radiologique 100 de l'art antérieur et a déjà été présentée en introduction.

La figure 2 représente schématiquement une vue en coupe d'une partie d'une cassette radiologique 10 selon l'invention. La cassette radiologique portable 10 comprend un détecteur numérique 11 de rayonnement ionisant sous forme d'un panneau plat. La cassette 10 comprend une embase 12 monobloc comprenant une première face principale 13, une deuxième face principale 14, opposée à la première face principale 13, l'embase 12 étant délimitée par quatre faces latérales 15, 16, 17, 18 (la face 16 est la face latérale arrière non visible sur cette vue en coupe et la face 18 est la face latérale avant non visible sur cette vue en coupe), l'embase 12 supportant le détecteur numérique 11 sur la première face principale 13. L'embase s'étend selon un plan (XY). Le détecteur numérique est donc superposé selon l'axe Z perpendiculaire au plan (XY) sur l'embase 12. La cassette 10 comprend en outre au moins une carte électronique 19 assurant la gestion du détecteur numérique 11. La carte électronique 19 est généralement positionnée sur la deuxième face principale 14 de l'embase, mais elle peut également être positionnée sur la première face principale 13 ou à une autre localisation de la cassette 10. Enfin, la cassette 10 comprend un boîtier 20 de protection mécanique, dans lequel sont disposés l'embase 12, le détecteur numérique 11 et la carte électronique 19. Le boîtier 20 comporte quatre faces latérales 25, 26, 27, 28 (la face 26 est la face latérale arrière non visible sur cette vue en coupe et la face 28 est la face latérale avant non visible sur cette vue en coupe), une face supérieure 23 et une face inférieure 24. Selon l'invention, la cassette radiologique portable 10 comprend au moins une pièce tridimensionnelle 30 (dans la suite, nous considérons, à titre d'exemple et de manière non limitative plusieurs pièces tridimensionnelles), chacune des pièces tridimensionnelles 30 étant associée à une des quatre faces latérales 15, 16, 17, 18 de l'embase 12. Chaque pièce tridimensionnelle 30 comprend une partie inférieure 31 reliée à l'embase 12 et enveloppant au moins partiellement la face latérale 15, 16, 17, 18 de l'embase 12 à laquelle la pièce tridimensionnelle 30 est associée. Comme détaillé ci-dessous, la partie inférieure 31 reliée à l'embase 12 peut envelopper au moins partiellement au moins une face latérale de l'embase 12 à laquelle la pièce tridimensionnelle 30 est associée. Elle peut par exemple en envelopper deux. Et chaque pièce tridimensionnelle 30 comprend une partie supérieure 32 s'étendant depuis la première face principale 13 de l'embase 12 jusqu'à la face supérieure 23 du boîtier 20.

Les pièces tridimensionnelles peuvent être assimilées à des absorbeurs tridimensionnels de choc dans les trois dimensions X, Y, Z.

Les pièces tridimensionnelles 30 peuvent être en élastomère, en polyuréthane, en élastomère thermoplastique, en polyamide et/ou en polyester ou tout autre matériau possédant des caractéristiques mécaniques similaires.

Les pièces tridimensionnelles garantissent les reprises d'efforts entre le panneau (ensemble formé par l'embase 12 et le détecteur numérique 11) et le boîtier 20 dans les trois directions. Les pièces tridimensionnelles permettent de filtrer les accélérations transmises à la technologie d'imagerie X (panneau) lors de chutes du détecteur.

Les pièces tridimensionnelles sont conçues de telle sorte que le calage en hauteur selon l'axe Z est réalisé entre les modules. Cela évite de transmettre les vibrations extérieures aux modules. Cela filtre la transmission des vibrations aux modules et permet une réduction significative des défauts dits « de microphonie ».

En outre, les propriétés du matériau utilisé, préférentiellement mais de manière non-limitative en élastomère, permettent également de limiter les jeux internes entre le panneau et le boîtier. Il en résulte une limitation des mouvements intérieurs qui seraient source de défauts lors de l'utilisation du produit (notamment déconnexions électriques, abrasion).

Grâce aux pièces tridimensionnelles, le positionnement des éléments de détection par rapport au marquage extérieur est garanti de manière plus précise.

Les possibilités de choix de matériau de ces pièces nous permettent d'envisager des procédés de fabrication par injection, et donc permettent de produire ces pièces avec de faibles coûts de revient. D'autres procédés de fabrication, par exemple la fabrication additive, sont aussi envisageables, comme discuté ci-dessous.

Les pièces tridimensionnelles jouent un rôle de calage de l'embase par rapport au boîtier à la fois dans le plan XY et aussi selon la hauteur Z. Une seule pièce permet de garantir isolation aux chocs selon les trois axes X, Y, Z. Les avantages qui en découlent sont une grande robustesse mécanique malgré les chutes de la cassette, les vibrations lors d'acquisition d'images, les manipulations brusques par l'utilisateur. Les pièces tridimensionnelles assurent une protection contre les sollicitations mécaniques occasionnelles ou répétées qui risquent de dégrader irréversiblement le produit ou d'altérer ses performances lors d'une utilisation en environnement perturbé. De plus, les pièces tridimensionnelles ne présentent pas un surcoût notable du détecteur.

La partie active interne des détecteurs numériques est constituée de pièces fragiles qui risquent de casser en cas de chute ou de choc violent de la cassette. Le cœur actif de la cassette (panneau + cartes électroniques) peut subir d'importantes déformations sous l'effet des ondes de choc créées par une chute ou un choc latéral violent sur l'enveloppe externe.

Un maintien souple ou élastique doit néanmoins assurer le positionnement de la zone image sensible par rapport à la référence mécanique externe du produit.

Ces détecteurs numériques plans sont également sensibles aux perturbations mécaniques venant du milieu extérieur (chocs, vibrations) pendant leur utilisation courante. Sans être destructives pour le produit, ces perturbations mécaniques sont susceptibles de créer des artefacts visibles dans les images radiologiques le plus souvent sous forme de lignes plus ou moins sombres qui apparaissent dans les images et dégradent fortement la qualité de celles-ci.

L'invention permet donc d'éviter de casser la partie active interne en cas de chute du produit (les hauteurs de chute étant typiquement de 80 cm à 120 cm), d'éviter les perturbations induites dans les images.

Dans un mode de réalisation (visible sur la partie droite de la figure 2), la partie inférieure 31 reliée à l'embase 12 s'étend depuis la deuxième face principale 14 de l'embase 12 jusqu'à la première face principale 13 de l'embase et en contact avec la face latérale du boîtier 20 en vis-à-vis. Dans ce mode de réalisation, la partie inférieure 31 prend en tenaille une portion de l'embase 12.

Dans un autre mode de réalisation (visible sur la partie gauche de la figure 2), la partie inférieure 31 reliée à l'embase 12 s'étend depuis la face latérale de l'embase 12 jusqu'à la première face principale 13 de l'embase et en contact avec la face latérale du boîtier 20 en vis-à-vis. Dans ce mode de réalisation, la partie inférieure 31 prend en tenaille une portion de l'embase 12.

Les pièces tridimensionnelles selon l'invention peuvent également prendre d'autres formes, par exemple des équerres, avec une partie inférieure reliée à l'embase.

La partie inférieure 31 des pièces tridimensionnelles 30 est préférentiellement collée à l'embase 12. Elle peut également être clipsée ou fixée à l'embase par tout autre moyen de fixation adapté.

La figure 3 représente schématiquement une vue d'une partie de la cassette numérique portable 10 selon l'invention sans boîtier 20 de protection mécanique.

La cassette radiologique 10 peut comprendre des premières pièces tridimensionnelles 30 et/ou des deuxièmes pièces tridimensionnelles 40. Les premières pièces tridimensionnelles 30 et les deuxièmes pièces tridimensionnelles 40 comprennent une partie inférieure 31 et une partie supérieure 32. Les premières pièces tridimensionnelles 30 ont leur partie supérieure 32 qui s'étend depuis leur partie inférieure 31 et jusqu'à une certaine hauteur selon l'axe Z correspondant à la hauteur sous boîtier, c'est-à-dire sous la face 23 du boîtier 20. La partie supérieure vise à reprendre les efforts selon Z.

Les deuxièmes pièces tridimensionnelles 40 se distinguent des premières pièces tridimensionnelles 30 par leur partie inférieure 41. La cassette radiologique portable 10 comprend généralement un (ou plusieurs) circuit souple 60 pouvant contenir un composant électronique. La partie inférieure 41 des pièces tridimensionnelles 40 comprend au moins un évidement 44 destiné à loger le circuit souple 60.

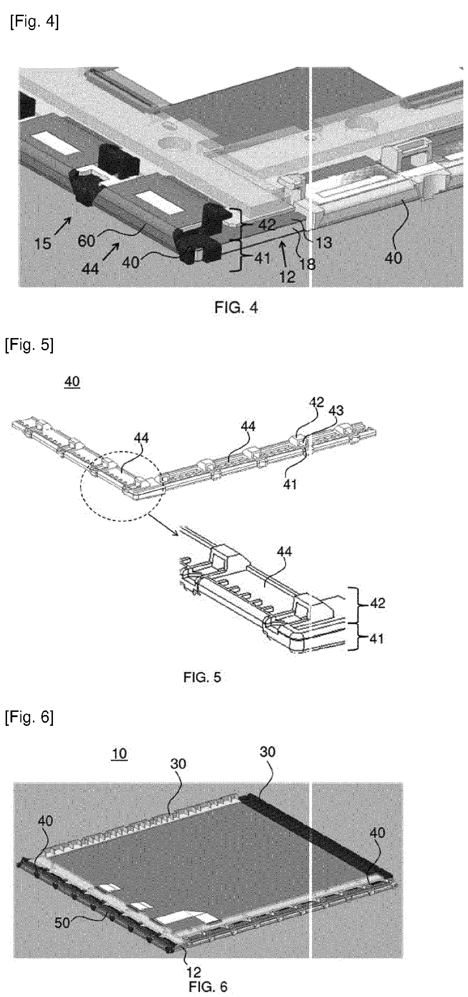

La figure 4 représente schématiquement un agrandissement de la vue de la cassette numérique portable 10 selon l'invention sans boîtier 20 présentée à la figure 3. Comme on le voit un peu plus en détail, le circuit souple 60 est positionné dans l'évidement 44 de la partie inférieure 41. Ce positionnement permet au circuit souple d'être bien calé en position. En cas de choc latéral, la partie inférieure 41 peut s'écraser légèrement pour absorber les efforts.

La partie supérieure 42 d'au moins une des pièces tridimensionnelles 40 peut se terminer par une forme complémentaire à la face supérieure 23 du boîtier 20. Autrement dit, la partie supérieure 42 se termine dans sa partie haute par une combinaison d'évidements et/ou protubérances de forme complémentaire à la forme de la face 23 pour épouser les formes intérieures du boîtier 20.

La partie inférieure 41 d'au moins une des pièces tridimensionnelles 40 (tout comme cela est aussi vrai pour la partie inférieure 31 d'une pièce 30) peut envelopper partiellement une face latérale 18 adjacente à la face latérale 15 de l'embase 12 à laquelle la pièce tridimensionnelle est associée. Autrement dit, la partie inférieure de la pièce tridimensionnelle peut envelopper un coin de l'embase.

La figure 5 représente une pièce tridimensionnelle 40 pour une cassette numérique portable 10 selon l'invention. Comme expliqué précédemment, la pièce tridimensionnelle 40 est destinée à coopérer avec :
- une embase 12 monobloc comprenant une première face principale 13, une deuxième face principale 14, opposée à la première face principale 13, l'embase 12 étant délimitée par quatre faces latérales 15, 16, 17, 18, l'embase 12 étant apte à supporter un détecteur numérique 11 sur la première face principale 13 et une carte électronique,
- un boîtier 20 de protection mécanique, l'embase 12, le détecteur numérique 11 et la carte électronique 19 étant destinés à être disposés dans le boîtier 20 de protection mécanique, le boîtier 20 comportant quatre faces latérales 25, 26, 27, 28, une face supérieure 23 et une face inférieure 24.

Selon l'invention, la pièce tridimensionnelle 40 comprend une partie inférieure 41 reliée à l'embase 12 et enveloppant au moins partiellement une face latérale 15, 16, 17, 18 de l'embase 12 ; et une partie supérieure 42 s'étendant depuis la première face principale 13 de l'embase 12 jusqu'à la face supérieure 23 du boîtier 20.

Sur cette figure, l'évidement 44 de la partie inférieure 41 des pièces tridimensionnelles 40 destiné à loger le circuit souple 60 est mis en évidence.

On peut noter que la pièce tridimensionnelle peut être associée à une face latérale de l'embase (comme présenté aux figures 3 et 4) ou bien elle peut être associée à plus de deux faces latérales, par exemple deux, comme on le voit sur la figure 5. Dans cette dernière variante, la partie inférieure 41 s'étend le long de deux bords latéraux de l'embase 12. Une telle pièce tridimensionnelle, en plus d'englober deux faces latérales de l'embase, permet de bien positionner les éléments par rapport au boîtier. Et une seul pièce tridimensionnelle suffit à absorber les chocs dans trois directions (X et Y par les parties inférieures 41 et Z par les parties supérieures 42).

La partie supérieure 42 d'au moins une des pièces tridimensionnelles 40 comprend au moins une ouverture 43 selon un axe sensiblement perpendiculaire à la face latérale de l'embase 12 à laquelle la pièce tridimensionnelle 40 est associée. Le rôle de cette ouverture 43 est expliqué au moyen de la figure suivante.

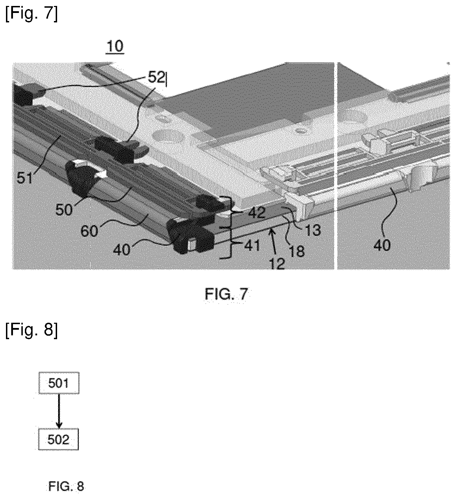

La figure 6 représente schématiquement une vue d'un autre mode de réalisation de la cassette numérique portable 10 selon l'invention sans boîtier 20, en lien avec les deuxièmes pièces tridimensionnelles 40. La vue présentée à la figure 6 est identique à la vue présentée à la figure 3. Dans cette vue, la cassette 10 comprend en outre un peigne 50 qui est destiné à coopérer avec une pièce tridimensionnelle 40, comme détaillée à la figure 7, pour assurer le maintien en position du circuit souple 60 dans l'évidement et venir le plaquer contre la partie inférieure 41 de la pièce tridimensionnelle.

La figure 7 représente schématiquement un agrandissement de la vue de la cassette numérique portable 10 selon l'invention sans boîtier 20 présentée à la figure 6. Comme on le voit un peu plus en détail, la cassette 10 comprend un peigne 50 comprenant une branche 51 s'étendant sensiblement parallèlement à la première face principale 13 de l'embase 12 et en contact sur la partie inférieure 41 de la pièce tridimensionnelle 40, la branche 51 étant pourvue d'au moins une dent 52 s'étendant sensiblement perpendiculairement à la branche 51, la au moins une dent 52 étant configurée pour coopérer avec la pièce tridimensionnelle 40 de sorte à immobiliser la branche 51 contre le circuit souple (60).

La au moins une dent 52 est insérée dans l'ouverture 43 de la partie supérieure 42 de la pièce tridimensionnelle 40 et configurée pour bloquer le peigne 50 en translation dans un plan sensiblement parallèle à l'embase 12. Le peigne 50 est bloqué en translation dans le plan XY et, par l'insertion des dents 52 dans les ouvertures 43, selon l'axe Z. Le peigne 50 plaque les circuits souples 60 vers l'embase, contre la partie inférieure de la pièce tridimensionnelle 40. Le peigne garantit le bon positionnement des circuits souples 60 qui doivent impérativement être maintenus très précisément par rapport à l'embase. On peut également noter que la partie inférieure 41 de la pièce 40 peut dépasser très légèrement (de l'ordre de quelques dixièmes de millimètre) du circuit souple. Autrement dit, l'épaisseur latérale de la partie inférieure 41 est supérieure à l'épaisseur du circuit souple qui peut éventuellement dépasser de l'embase au niveau de la face latérale de l'embase. Cette configuration contribue au maintien en position du circuit souple même en cas de chocs : la pièce tridimensionnelle est suffisamment souple pour s'écraser et assurer une portée homogène sur toute la longueur de la pièce tridimensionnelle.

Dans ce qui précède, seul un peigne 50 a été mentionné. L'invention peut s'appliquer avec un seul peigne, mais elle comprend avantageusement plusieurs peignes, comme représenté sur la figure 7.

Ainsi, en plus de leur rôle d'absorbeur tridimensionnel de chocs, la pièce tridimensionnelle 40 permet l'intégration d'un peigne, pièce de maintien des modules/circuits souples, isolé mécaniquement du boîtier. Cela contribue à l'amélioration de la filtration des vibrations extérieures vers les modules/circuits souples.

L'invention repose sur la pièce tridimensionnelle capable d'absorber des chocs et vibrations dans toutes les directions X, Y, Z et de protéger ainsi les détecteurs contre les risques de casse. La pièce tridimensionnelle constitue un filtre mécanique entre le boîtier de protection mécanique et le cœur actif du détecteur. Sa structure élastique permet de positionner la zone image sensible du détecteur numérique par rapport à la référence mécanique extérieure.

Un autre avantage de l'invention est que cette pièce tridimensionnelle est démontable et permet une réparation ou un échange facile du panneau et des cartes électroniques. Enfin, cette pièce tridimensionnelle contribue à la rigidité du panneau en limitant les courbures locales mal maîtrisées lors de la propagation des ondes de choc créées par un choc latéral.

L'invention concerne aussi un produit programme d'ordinateur, ledit programme d'ordinateur comprenant des instructions exécutables par ordinateur qui, lorsqu'elles sont exécutées par un processeur, amènent le processeur à commander un appareil de fabrication additive pour fabriquer la pièce tridimensionnelle 30 ou 40 telle que décrite précédemment.

La figure 8 représente schématiquement les étapes d'un procédé de fabrication de la pièce tridimensionnelle selon l'invention par fabrication additive. Le procédé de fabrication de la pièce tridimensionnelle 30, 40 par fabrication additive comprend une étape 501 qui consiste à obtenir un fichier électronique représentant une géométrie d'un produit dans lequel le produit est la pièce tridimensionnelle 30 ou 40; et une étape 502 de commande d'un appareil de fabrication additive pour fabriquer, sur une ou plusieurs étapes de fabrication additive, le produit selon la géométrie spécifiée dans le fichier électronique.

Des exemples selon la divulgation peuvent être formés en utilisant un procédé de fabrication additive. Un exemple courant de fabrication additive est l'impression 3D; cependant, d'autres méthodes de fabrication additive sont disponibles. Le prototypage rapide ou la fabrication rapide sont également des termes qui peuvent être utilisés pour décrire des procédés de fabrication additive. Tel qu'utilisé ici, «fabrication additive» se réfère généralement à des procédés de fabrication dans lesquels des couches successives de matériau(x) sont disposées les unes sur les autres pour «construire» couche par couche ou «fabriquer de manière additive», un composant tridimensionnel. Ceci est comparé à certains procédés de fabrication soustractifs (tels que le fraisage ou le perçage), dans lesquels le matériau est successivement retiré pour fabriquer la pièce. Les couches successives fusionnent généralement ensemble pour former un composant monolithique qui peut avoir une variété de sous-composants intégrés. En particulier, le procédé de fabrication peut permettre à un exemple de la divulgation d'être formé intégralement et inclure une variété de caractéristiques qui ne sont pas possibles lors de l'utilisation de procédés de fabrication antérieurs. Les procédés de fabrication additive décrits ici permettent la fabrication à n'importe quelle taille et forme appropriées avec diverses caractéristiques qui peuvent ne pas avoir été possibles en utilisant des procédés de fabrication antérieurs. La fabrication additive peut créer des géométries complexes sans utiliser aucun type d'outils, de moules ou de montages, et avec peu ou pas de déchets. Au lieu d'usiner des composants à partir de billettes solides de plastique ou de métal, dont une grande partie est découpée et jetée, le seul matériau utilisé dans la fabrication additive est ce qui est nécessaire pour façonner la pièce.

Les techniques de fabrication additive appropriées selon la présente divulgation comprennent, par exemple, la modélisation par dépôt fondu (Fused Deposition Modeling ou FDM), le frittage laser sélectif (Selective Laser Sintering ou SLS), l'impression 3D telle que par jet d'encre et laserjet, la stéréolithographie (Sterolithography ou SLA), le frittage laser sélectif direct (Sterolithography ou DSLS) , frittage par faisceau d'électrons (Electron Beam Sintering ou EBS), fusion par faisceau d'électrons (Electron Beam Sintering ou EBM), mise en forme de filet par laser (Laser Engineered Net Shaping ou LENS), fabrication additive de faisceau d'électrons (Electron Beam Additive Manufacturing ou EBAM), fabrication de forme de filet au laser (Laser Net Shape Manufacturing ou LNSM), dépôt direct de métal (Direct Metal Deposition ou DMD), traitement numérique de la lumière (Digital Light Processing ou DLP), traitement numérique continu de la lumière (Continuous Digital Light Processing ou CDLP), fusion laser sélective directe (Direct Selective Laser Melting ou DSLM), fusion laser sélective (Selective Laser Melting ou SLM), fusion laser directe des métaux (Direct Metal Laser Melting ou DMLM), frittage laser direct des métaux (Direct Metal Laser Sintering ou DMLS), projection de matière (Material Jetting ou MJ), projection de nanoparticules (NanoParticle Jetting ou NPJ), dépôt à la demande (Drop On Demand ou DOD), jet de liant (Binder Jetting ou BJ), Multi Jet Fusion (MJF), fabrication d'objets stratifiés (Laminated Object Manufacturing ou LOM) et autres procédés connus. Les procédés de fabrication additive décrits ici peuvent être utilisés pour former des composants en utilisant n'importe quel matériau approprié. Par exemple, le matériau peut être plastique, composite, polymère, époxy, résine photopolymère ou tout autre matériau approprié qui peut être sous forme solide, liquide, poudre, feuille, fil ou toute autre forme appropriée ou combinaisons de ceux-ci. Plus spécifiquement, selon des modes de réalisation exemplaires du présent objet, les composants fabriqués de manière additive décrits ici peuvent être formés en partie, en totalité ou dans une certaine combinaison de matériaux. Ces matériaux sont des exemples de matériaux appropriés pour une utilisation dans des procédés de fabrication additive qui peuvent être appropriés pour la fabrication d'exemples décrits ici.

Comme indiqué ci-dessus, le procédé de fabrication additive décrit ici permet à un seul composant d'être formé à partir de plusieurs matériaux. Ainsi, les exemples décrits ici peuvent être formés à partir de tout mélange approprié des matériaux ci-dessus. Par exemple, un composant peut comprendre plusieurs couches, segments ou pièces qui sont formés en utilisant différents matériaux, procédés et/ou sur différentes machines de fabrication additive. De cette manière, des composants peuvent être construits qui ont des matériaux et des propriétés de matériaux différents pour répondre aux exigences de toute application particulière. De plus, bien que les composants décrits ici soient entièrement construits par des procédés de fabrication additive, il doit être apprécié que dans des modes de réalisation alternatifs, tout ou partie de ces composants peuvent être formés par moulage, usinage et/ou tout autre procédé de fabrication approprié. En effet, toute combinaison appropriée de matériaux et de procédés de fabrication peut être utilisée pour former ces composants. Les procédés de fabrication additive fabriquent généralement des composants sur la base d'informations tridimensionnelles (3D), par exemple un modèle informatique tridimensionnel (ou fichier de conception), du composant. En conséquence, les exemples décrits ici comprennent non seulement des produits ou composants tels que décrits ici, mais également des procédés de fabrication de tels produits ou composants via la fabrication additive et des logiciels, micrologiciels ou matériels informatiques pour contrôler la fabrication de tels produits via la fabrication additive.

La structure d'une ou plusieurs parties du produit peut être représentée numériquement sous la forme d'un fichier de conception. Un fichier de conception, ou fichier de conception assistée par ordinateur (CAO), est un fichier de configuration qui code une ou plusieurs configurations de surface ou volumétrique de la forme du produit. Autrement dit, un fichier de conception représente la disposition géométrique ou la forme du produit. Les fichiers de conception peuvent prendre n'importe quel format de fichier maintenant connu ou développé ultérieurement. Par exemple, les fichiers de conception peuvent être au format Stereolithography ou «Standard Tessellation Language» (.stl) qui a été créé pour les programmes de stéréolithographie CAO de 3D Systems, ou au format Additive Manufacturing File (.amf), qui est une société américaine de mécanique Norme des ingénieurs (ASME) qui est un format basé sur un langage de balisage extensible (XML) conçu pour permettre à tout logiciel de CAO de décrire la forme et la composition de tout objet tridimensionnel à fabriquer sur n'importe quelle imprimante de fabrication additive. D'autres exemples de formats de fichiers de conception incluent les fichiers AutoCAD (.dwg), les fichiers Blender (.blend), les fichiers Parasolid (.x_t), les fichiers 3D Manufacturing Format (.3mf), les fichiers Autodesk (3ds), les fichiers Collada (.dae) et Fichiers Wavefront (.obj), bien que de nombreux autres formats de fichiers existent. Les fichiers de conception peuvent être produits à l'aide d'un logiciel de modélisation (par exemple, la modélisation CAO) et/ou en scannant la surface d'un produit pour mesurer la configuration de la surface du produit.

Une fois obtenu, un fichier de conception peut être converti en un ensemble d'instructions exécutables par ordinateur qui, une fois exécutées par un processeur, amènent le processeur à contrôler un appareil de fabrication additive pour produire un produit selon la disposition géométrique spécifiée dans le fichier de conception. La conversion peut convertir le fichier de conception en tranches ou couches qui doivent être formées séquentiellement par l'appareil de fabrication additive. Les instructions (également connues sous le nom de code géométrique ou «code G») peuvent être calibrées en fonction de l'appareil de fabrication additive spécifique et peuvent spécifier l'emplacement précis et la quantité de matériau à former à chaque étape du processus de fabrication. Comme discuté ci-dessus, la formation peut se faire par dépôt, par frittage ou par toute autre forme de procédé de fabrication additive. Le code ou les instructions peuvent être traduits entre différents formats, convertis en un ensemble de signaux de données et transmis, reçus sous la forme d'un ensemble de signaux de données et convertis en code, stockés, etc., si nécessaire. Les instructions peuvent être une entrée du système de fabrication additive et peuvent provenir d'un concepteur de pièces, d'un fournisseur de propriété intellectuelle (PI), d'une société de conception, de l'opérateur ou du propriétaire du système de fabrication additive, ou d'autres sources. Un système de fabrication additive peut exécuter les instructions pour fabriquer le produit en utilisant l'une quelconque des technologies ou procédés décrits ici. Les fichiers de conception ou les instructions exécutables par ordinateur peuvent être stockés dans un support de stockage lisible par ordinateur (transitoire ou non) (par exemple, mémoire, système de stockage, etc.) stockant un code, ou des instructions lisibles par ordinateur, représentatives du produit à fabriquer. Comme noté, le code ou les instructions lisibles par ordinateur définissant le produit qui peut être utilisé pour générer physiquement l'objet, lors de l'exécution du code ou des instructions par un système de fabrication additive. Par exemple, les instructions peuvent inclure un modèle 3D défini avec précision du produit et peuvent être générées à partir de l'un des nombreux systèmes logiciels de conception assistée par ordinateur (CAO) bien connus tels qu'AutoCAD^{®}, TurboCAD^{®}, DesignCAD 3D Max, etc. En variante, un modèle ou prototype du composant peut être scanné pour déterminer les informations tridimensionnelles du composant.

En conséquence, en commandant un appareil de fabrication additive selon les instructions exécutables par ordinateur, l'appareil de fabrication additive peut être chargé d'imprimer une ou plusieurs parties du produit. Ceux-ci peuvent être imprimés sous forme assemblée ou non assemblée. Par exemple, différentes sections du produit peuvent être imprimées séparément (sous forme de kit de pièces non assemblées), puis assemblées. En variante, et de manière préférentielle, les différentes pièces peuvent être imprimées sous forme assemblée. À la lumière de ce qui précède, les modes de réalisation comprennent des procédés de fabrication par fabrication additive. Cela comprend les étapes d'obtention d'un fichier de conception représentant le produit et l'instruction d'un appareil de fabrication additive de fabriquer le produit sous une forme assemblée ou non assemblée selon le fichier de conception. L'appareil de fabrication additive peut comprendre un processeur qui est configuré pour convertir automatiquement le fichier de conception en instructions exécutables par ordinateur pour contrôler la fabrication du produit. Dans ces modes de réalisation, le fichier de conception lui-même peut provoquer automatiquement la production du produit une fois entré dans le dispositif de fabrication additive. En conséquence, dans ce mode de réalisation, le fichier de conception lui-même peut être considéré comme des instructions exécutables par ordinateur qui amènent l'appareil de fabrication additive à fabriquer le produit. En variante, le fichier de conception peut être converti en instructions par un système informatique externe, les instructions exécutables par ordinateur résultantes étant fournies au dispositif de fabrication additive. Compte tenu de ce qui précède, la conception et la fabrication des implémentations du sujet et les opérations décrites dans cette spécification peuvent être réalisées à l'aide de circuits électroniques numériques, ou dans un logiciel, un micrologiciel ou du matériel informatique, y compris les structures décrites dans cette spécification et leurs équivalents structurels, ou en combinaisons d'un ou plusieurs d'entre eux. Par exemple, le matériel peut inclure des processeurs, des microprocesseurs, des circuits électroniques, des composants électroniques, des circuits intégrés, etc. Les implémentations du sujet décrit dans cette spécification peuvent être réalisées en utilisant un ou plusieurs programmes informatiques, c'est-à-dire un ou plusieurs modules d'instructions de programmes informatiques, codés sur un support de stockage informatique pour exécution par ou pour commander le fonctionnement d'un appareil de traitement de données. En variante ou en plus, les instructions de programme peuvent être codées sur un signal propagé généré artificiellement, par exemple, un signal électrique, optique ou électromagnétique généré par machine pour coder des informations à transmettre à un appareil récepteur approprié pour exécution par un appareil de traitement de données. Un support de stockage informatique peut être, ou être inclus dans, un dispositif de stockage lisible par ordinateur, un substrat de stockage lisible par ordinateur, une matrice ou un dispositif de mémoire à accès aléatoire ou série, ou une combinaison d'un ou plusieurs d'entre eux. De plus, alors qu'un support de stockage informatique n'est pas un signal propagé, un support de stockage informatique peut être une source ou une destination d'instructions de programme informatique codées dans un signal propagé artificiellement généré. Le support de stockage informatique peut également être, ou être inclus dans, un ou plusieurs composants ou supports physiques séparés (par exemple, plusieurs CD, disques ou autres dispositifs de stockage). Bien que la technologie de fabrication additive soit décrite ici comme permettant la fabrication d'objets complexes en construisant des objets point par point, couche par couche, typiquement dans une direction verticale, d'autres procédés de fabrication sont possibles et dans le cadre du présent sujet. Par exemple, bien que la description ici se réfère à l'addition de matériau pour former des couches successives, l'homme du métier appréciera que les procédés et structures décrits ici peuvent être mis en pratique avec n'importe quelle technique de fabrication additive ou autre technologie de fabrication. On peut également citer le moulage par injection ou la fabrication par coulée d'élastomère basse pression.

L'invention s'applique principalement à la réalisation de capteurs d'images pour la radiologie et plus particulièrement aux capteurs de type "portable" qui sont régulièrement soumis à des chocs ou susceptibles de tomber lorsqu'ils sont manipulés.

## Revendications

1. Cassette radiologique portable (10) comprenant :
- Un détecteur numérique (11) de rayonnement ionisant sous forme d'un panneau plat ;
- Une embase (12) monobloc comprenant une première face principale (13), une deuxième face principale (14), opposée à la première face principale (13), l'embase (12) étant délimitée par quatre faces latérales (15, 16, 17, 18), l'embase (12) supportant le détecteur numérique (11) sur la première face principale (13) ;
- une carte électronique (19) assurant la gestion du détecteur numérique (11) ;
- un boîtier (20) de protection mécanique, dans lequel sont disposés l'embase (12), le détecteur numérique (11) et la carte électronique (19), le boîtier (20) comportant quatre faces latérales (25, 26, 27, 28), une face supérieure (23) et une face inférieure (24);
- au moins une pièce tridimensionnelle (30, 40), chacune des au moins une pièce tridimensionnelle (30, 40) étant associée à au moins une des quatre faces latérales (15, 16, 17, 18) de l'embase (12), chaque pièce tridimensionnelle (30, 40) comprenant :
- une partie inférieure (31, 41) reliée à l'embase (12) et enveloppant au moins partiellement la au moins une face latérale (15, 16, 17, 18) de l'embase (12) à laquelle la pièce tridimensionnelle (30, 40) est associée;
- une partie supérieure (32, 42) s'étendant depuis la première face principale (13) de l'embase (12) jusqu'à la face supérieure (23) du boîtier (20) ;
en ce que la cassette radiologique portable comprend un circuit souple (60) ;
la partie inférieure (41) d'au moins une des pièces tridimensionnelles (40) comprenant au moins un évidement (44) destiné à loger le circuit souple (60) ;
la partie supérieure (42) d'au moins une des pièces tridimensionnelles (40) comprenant au moins une ouverture (43) selon un axe sensiblement perpendiculaire à la face latérale de l'embase (12) à laquelle la pièce tridimensionnelle (40) est associée ;
caractérisée et en ce que la cassette radiologique portable comprend un peigne (50) comprenant une branche (51) s'étendant sensiblement parallèlement à la première face principale (13) de l'embase (12) et en contact sur la partie inférieure (41) de la pièce tridimensionnelle (40), la branche (51) étant pourvue d'au moins une dent (52) s'étendant sensiblement perpendiculairement à la branche (51), la au moins une dent (52) étant configurée pour coopérer avec la pièce tridimensionnelle (40) de sorte à immobiliser la branche (51) contre le circuit souple (60).

2. Cassette radiologique portable (10) selon la revendication 1, dans laquelle la partie inférieure (31, 41) d'au moins une des pièces tridimensionnelles (30, 40) enveloppe partiellement une face latérale adjacente à la face latérale de l'embase (12) à laquelle la pièce tridimensionnelle (30, 40) est associée.

3. Cassette radiologique portable (10) selon la revendication 1 ou 2, dans laquelle la au moins une dent (52) est insérée dans l'ouverture (43) de la partie supérieure (42) de la pièce tridimensionnelle (40) et configurée pour bloquer le peigne (50) en translation dans un plan sensiblement parallèle à l'embase (12).

4. Cassette radiologique portable (10) selon l'une des revendications 1 à 3, dans laquelle la partie supérieure d'au moins une des pièces tridimensionnelles se termine par une forme complémentaire à la face supérieure (23) du boîtier (20).

5. Cassette radiologique portable (10) selon l'une des revendications 1 à 4, dans laquelle la au moins une pièce tridimensionnelle (30, 40) est en élastomère, en polyuréthane, en élastomère thermoplastique, en polyamide et/ou en polyester.

6. Cassette radiologique portable (10) selon l'une des revendications 1 à 5, dans laquelle la partie inférieure (31, 41) de la au moins une pièce tridimensionnelle (30, 40) est collée à l'embase (12).

## Patentansprüche

1. Tragbare radiologische Kassette (10), umfassend:
- einen digitalen Detektor (11) für ionisierende Strahlung in Form einer flachen Platte;
- einen einteiligen Sitz (12), der eine erste Hauptfläche (13) und eine zweite Hauptfläche (14) umfasst, die der ersten Hauptfläche (13) gegenüberliegt, wobei der Sitz (12) durch vier Seitenflächen (15, 16, 17, 18) begrenzt wird, wobei der Sitz (12) den digitalen Detektor (11) auf der ersten Hauptfläche (13) trägt;
- eine elektronische Karte (19), die die Verwaltung des digitalen Detektors (11) gewährleistet;
- ein Gehäuse (20) zum mechanischen Schutz, in dem der Sitz (12), der digitale Detektor (11) und die elektronische Karte (19) angeordnet sind, wobei das Gehäuse (20) vier Seitenflächen (25, 26, 27, 28), eine obere Fläche (23) und eine untere Fläche (24) aufweist;
- mindestens ein dreidimensionales Teil (30, 40), wobei jedes von mindestens einem dreidimensionalen Teil (30, 40) mindestens einer der vier Seitenflächen (15, 16, 17, 18) des Sitzes (12) zugeordnet ist, wobei jedes dreidimensionale Teil (30, 40) umfasst:
- einen unteren Teil (31, 41), der mit dem Sitz (12) verbunden ist und mindestens teilweise die mindestens eine Seitenfläche (15, 16, 17, 18) des Sitzes (12) umgibt, dem das dreidimensionale Teil (30, 40) zugeordnet ist;
- einen oberen Teil (32, 42), der sich von der ersten Hauptfläche (13) des Sitzes (12) bis zur oberen Fläche (23) des Gehäuses (20) erstreckt;
dass die tragbare radiologische Kassette eine flexible Schaltung (60) umfasst;
wobei der untere Teil (41) mindestens eines der dreidimensionalen Teile (40) mindestens eine Aussparung (44) umfasst, die zur Aufnahme der flexiblen Schaltung (60) bestimmt ist;
wobei der obere Teil (42) mindestens eines der dreidimensionalen Teile (40) mindestens eine Öffnung (43) entlang einer Achse umfasst, die im Wesentlichen senkrecht zur Seitenfläche des Sitzes (12) verläuft, dem das dreidimensionale Teil (40) zugeordnet ist;
**dadurch gekennzeichnet, dass** die tragbare radiologische Kassette einen Kamm (50) umfasst, der einen Ast (51) umfasst, der sich im Wesentlichen parallel zur ersten Hauptfläche (13) des Sitzes (12) erstreckt und auf dem unteren Teil (41) des dreidimensionalen Teils (40) in Kontakt steht, wobei der Ast (51) mit mindestens einer Zinke (52) versehen ist, die sich im Wesentlichen senkrecht zum Ast (51) erstreckt, wobei die mindestens eine Zinke (52) konfiguriert ist, um mit dem dreidimensionalen Teil (40) derart zusammenzuwirken, dass der Ast (51) gegen die flexible Schaltung (60) immobilisiert wird.

2. Tragbare radiologische Kassette (10) nach Anspruch 1, wobei der untere Teil (31, 41) mindestens eines der dreidimensionalen Teile (30, 40) teilweise eine Seitenfläche umgibt, die an die Seitenfläche des Sitzes (12) angrenzt, dem das dreidimensionale Teil (30, 40) zugeordnet ist.

3. Tragbare radiologische Kassette (10) nach Anspruch 1 oder 2, wobei die mindestens eine Zinke (52) in die Öffnung (43) des oberen Teils (42) des dreidimensionalen Teils (40) eingeführt und konfiguriert ist, um den Kamm (50) in einer zum Sitz (12) im Wesentlichen parallelen Ebene in der Translation zu blockieren.

4. Tragbare radiologische Kassette (10) nach Anspruch 1 bis 3, wobei der obere Teil mindestens eines der dreidimensionalen Teile zu einer Form endet, die zur oberen Fläche (23) des Gehäuses (20) komplementär ist.

5. Tragbare radiologische Kassette (10) nach Anspruch 1 bis 4, wobei das mindestens eine dreidimensionale Teil (30, 40) aus Elastomer, aus Polyurethan, aus thermoplastischem Elastomer, aus Polyamid und/oder aus Polyester besteht.

6. Tragbare radiologische Kassette (10) nach einem der Ansprüche 1 bis 5, wobei der untere Teil (31, 41) des mindestens einen dreidimensionalen Teils (30, 40) an den Sitz (12) geklebt ist.

## Claims

1. Portable radiological cartridge (10) comprising:
- a digital detector (11) for ionizing radiation in the form of a flat panel;
- a single-piece base (12) comprising a first main face (13), a second main face (14) opposite the first main face (13), the base (12) being delimited by four lateral faces (15, 16, 17, 18), the base (12) supporting the digital detector (11) on the first main face (13);
- an electronic card (19) which ensures control of the digital detector (11);
- a housing (20) for mechanical protection, in which the base (12), the digital detector (11) and the electronic card (19) are arranged, the housing (20) comprising four lateral faces (25, 26, 27, 28), an upper face (23) and a lower face (24);
- at least one three-dimensional component (30, 40), each of the at least one three-dimensional component(s) (30, 40) being associated with at least one of the four lateral faces (15, 16, 17, 18) of the base (12), each three-dimensional component (30, 40) comprising:
- a lower portion (31, 41) which is connected to the base (12) and which at least partially surrounds the at least one lateral face (15, 16, 17, 18) of the base (12) with which the three-dimensional component (30, 40) is associated;
- an upper portion (32, 42) which extends from the first main face (13) of the base (12) as far as the upper face (23) of the housing (20);
in that the portable radiological cartridge comprises a flexible circuit (60);
the lower portion (41) of at least one of the three-dimensional components (40) comprising at least one recess (44) which is intended to accommodate the flexible circuit (60);
the upper portion (42) of at least one of the three-dimensional components (40) comprising at least one opening (43) along an axis substantially perpendicular to the lateral face of the base (12) with which the three-dimensional component (40) is associated;
**characterized in that** the portable radiological cartridge comprises a comb (50) which comprises a branch (51) which extends substantially parallel with the first main face (13) of the base (12) and is in contact with the lower portion (41) of the three-dimensional component (40), the branch (51) being provided with at least one tooth (52) which extends substantially perpendicularly to the branch (51), the at least one tooth (52) being configured to cooperate with the three-dimensional component (40) in order to fix the branch (51) against the flexible circuit (60).

2. Portable radiological cartridge (10) according to claim 1, wherein the lower portion (31, 41) of at least one of the three-dimensional components (30, 40) partially surrounds a lateral face adjacent to the lateral face of the base (12) with which the three-dimensional component (30, 40) is associated.

3. Portable radiological cartridge (10) according to claim 1 or 2, wherein the at least one tooth (52) is inserted into the opening (43) of the upper portion (42) of the three-dimensional component (40) and is configured to block the comb (50) in terms of translation in a plane substantially parallel with the base (12).

4. Portable radiological cartridge (10) according to any one of claims 1 to 3, wherein the upper portion of at least one of the three-dimensional components terminates with a shape which complements the upper face (23) of the housing (20).

5. Portable radiological cartridge (10) according to any one of claims 1 to 4, wherein the at least one three-dimensional component (30, 40) is made of elastomer material, polyurethane, thermoplastic elastomer material, polyamide and/or polyester.

6. Portable radiological cartridge (10) according to any one of claims 1 to 5, wherein the lower portion (31, 41) of the at least one three-dimensional component (30, 40) is adhesively bonded to the base (12).
